# EUROPEAN PATENT SPECIFICATION

(11) **EP 0 512 939 B1**
(45) Date of publication and mention of the grant of the patent: **25.08.1999**
(21) Application number: 92500056.4
(22) Date of filing: 08.05.1992
(51) Int. Cl.: C07D 235/30, A61K 31/415, A61K 31/495

(54) **2-piperazinylbenzimidazole derivatives**
2-Piperazinylbenzimidazolderivate
Dérivés de pipérazinyl-2-benzimidale

(30) Priority: 10.05.1991 ES 9101147
(43) Date of publication of application: 11.11.1992
(73) Proprietor: FABRICA ESPANOLA DE PRODUCTOS QUIMICOS Y FARMACEUTICOS, S.A. (FAES), E-48940 Leioa-Lamiaco (Vizcaya) (ES)
(72) Inventor: Orjales-Venero, Aurelio, Neguri (Vizcaya) (ES); Garcia-Dominguez, Neftali, E-48990 Guecho (Vizcaya) (ES); Rubio-Royo, Victor, E-48990 Guecho (Vizcaya) (ES); Rodes-Solanes, Rosa, E-48990 Guecho (Vizcaya) (ES)
(74) Representative: Blumbach, Kramer & Partner GbR

(56) References cited:
- EP-A- 0 079 545
- WO-A-91/18904
- CHEMICAL ABSTRACTS, vol. 84, no. 7, 16 February 1976, Columbus, Ohio, US; abstract no. 44060H, KODAMA,TSUTOMU; A.O.: '2-(4-Substituted piperazino)-benzimidazoles.' page 497 ;column 1 ;
- CHEMICAL ABSTRACTS, vol. 111, no. 5, 31 July 1989, Columbus, Ohio, US; abstract no. 33121D, IEMURA, RYUICHI; A.O.: 'Quantitative structure-activity relationships of H1-antihistaminic benzimidazole derivatives.' page 14 ;column 2 ;
- JOURNAL OF MEDICINAL CHEMISTRY. vol. 29, no. 7, July 1986, WASHINGTON US pages 1178 - 1183; RYUICHI IEMURA, A.O.: 'Synthesis of 2-(4-Substituted-1-piperazinyl)benzimidazoles as H1-Antihistaminic Agents.'
- JOURNAL OF MEDICINAL CHEMISTRY. vol. 35, no. 4, 21 February 1992, WASHINGTON US pages 705 - 716; JOHN W. ELLINGBOE, A.O.: 'Class III Antiarrhythmic Activity of Novel Substituted 4-[(Methylsulfonyl)amino]benzamides and Sulfonamides.'

## Description

The present invention relates to new 2-piperazinylbenzimidazole derivatives of formula (I), and their addition salts with pharmaceutically acceptable acids. 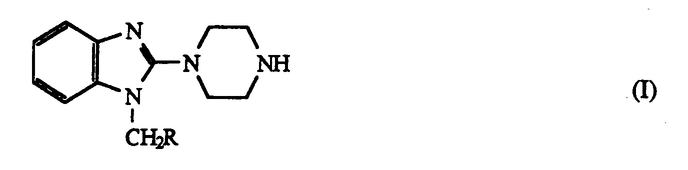 In formula (I), R represents a hydrogen atom, a linear or branched alkyl group having 1 to 3 carbon atoms or a phenyl group, substituted or not by a halogen in position 4. The halogen is fluorine, chlorine, bromine or iodine. Likewise comprised in this invention are the addition salts of these compounds with inorganic acids, such as hydrochloric, hydrobromic, phosphoric and sulphuric acids, and organic acids such as acetic, fumaric, tartaric, oxalic and benzoic acids.

Specifically, the invention relates to 2-Piperazinylbenzimidazole derivatives according to the above formula (I), namely
- 1-Methyl-2-(1-piperazinyl-)1H-benzimidazole;
- 1-Phenylmethyl-2-(1-piperazinyl-)1H-benzimidazole;
- 1-(4-Fluorophenylmethyl-)2-(1-piperazinyl-)1H-benzimidazole;
- 1-Ethyl-2-(1-piperazinyl-)1H-benzimidazole; and
- 1-Propyl-2-(1-piperazinyl-)1H-benzimidazole.

The invention furthermore relates to 2-Piperazinylbenzimidazole derivatives according to the above formula (I) for use as antagonists to the action of serotonin at the 5HT₃ receptors level.

The invention additionally relates to 2-Piperazinylbenzimidazole derivatives according to the above formula (I) for use as a medicament.

In addition, the invention relates to 2-Piperazinylbenzimidazole derivatives according to the above formula (I) for use as a medicament for preventing vomit.

The invention also relates to 2-Piperazinylbenzimidazole derivatives according to the above formula (I) for use as a medicament useful in the prophylaxis and treatment of migraine.

The invention still further relates to 2-Piperazinylbenzimidazole derivatives according to the above formula (I) for use as a medicament useful in the prophylaxis and treatment of anxiety.

Finally, the invention relates to 2-Piperazinylbenzimidazole derivatives according to the above formula (I) for use as a medicament useful in the prophylaxis and treatment of neuralgic disorders.

The Document "Chem. Abstracts, vol. 84, 1976, p. 497, no. 44060h" which is an Abstract of the published Japanese Patent Application JP-A 50-126,682 discloses compounds of the general formula (I) wherein the derivatives have an alkyl residue in the 4-position of the piperazyl residue; hydrogen is no substituent of the piperazyl residue in the 4-position.

The Document "Chem. Abstracts, vol. 111, 1989, p. 14 , no. 33121d refers to a scientific article by R. Iemura et al. in "Chem. Pharm. Bull. 37 (4) (1989), 967 et seq." and discloses chemical compounds of the general formula (I) having a hydrogen atom as the substituent in the 4-position of the piperazyl residue. Howver, there are in all cases features distinguishing the compounds of said document from the compounds of the present invention by at least one structural feature.

Document "Journal of Medicinal Chemistry, vol. 29, 1986, no. 7, p. 1177 - 83 dicloses details on the synthesis of the compounds of the general formula (I) two of which bear a hydrogen atom as the substituent in the 4-position of the piperazyl residue. However, there are in both cases features distinguishing the compounds of said document from the compounds of the present application.

The pharmacological activities of the compounds mentioned herein become clear by testing with animals using well established pharmacological processes. Two of the test made are mentioned to illustrate this:
a) Wistar rats of both sexes, weighing 180 to 220 g, were anaethesized with urethane (1.25 g/kg, i.p.), subsequently subjected to trachea, carotid artery and jugular vein cannulation, with spontaneous breathing and a rectal temperature of 37-38^{º}C. On establishing the arterial pressure parameters (AP) and cardiac frequency (CF), the Bezold-Jarisch reflex was induced by means of a fast intravenous injection of 80 µg/kg of the serotonin-creatinine sulphate complex. Ten minutes later, and upon restoring the CF and AP to constant levels, the products claimed herein injected intravenously and after five minutes intravenous injection of the serotonin-creatinine sulphate compound was repeated, quantifying inhibition of the Bezold-Jarisch reflex. All the compounds tested proved to be active when administered intravenously within the range lying between 0,3 and 10 µg/kg, therefore proving to be antagonist of the serotonin 5HT₃ receptors.
b) The inhibition of cisplatinum induced vomit in a conscious dog was studied. The products were administered intravenously 30 minutes before and 2 hours after the cisplatinum (3 mg/kg intravenously, 1 ml/kg). The number of emetic episodes were counted for five hours after administering the cisplatinum. The products hereof were more active than methochloramide in preventing cisplatinum induced vomit.
The results of these tests show that the compounds of the invention antagonize the action of serotonin at the 5HT₃ receptors level and are therefore appropriate for preventing vomit induced by anticancerous agents, such as cisplatinum and radiations, and are potentially useful in prophylaxis and treatment of migraine, anxiety and other neuralgic disorders.

The following examples provide more details of the inventions, without same being in any way confined thereto.

### Example 1

### Preparation of 1-Methyl-2-(1-piperazinyl)-1H-benzimidazole.

A mixture of 5 g (30 mmol) of 2-Chloro-1-methyl-1H-benzimidazole and 12.9 g (150 mmol) of anhydrous piperazine was heated at 125 ^{º}C for 15 minutes. The reagent mixture was provided with a 10% NaOH aqueous solution and extracted with dichloromethane (3 x 20 ml). The organic extracts obtained were dried on anhydrous sodium sulphate. After distilling the solvent in a vacuum, an oil was obtained that upon treatment with ether provided 4.5 g (69% yield) of a white solid, characterized as 1-Methyl-2-(1-piperazinyl)
- 1H-benzimidazole, with a melting point of 80 to 82°C. The following compounds were similarly prepared:
- 1-Phenylmethyl-1-(1-piperazinyl)-1H-benzimidazole. MP: 125 to 127°C.
- 1-(4-Fluorophenylmethyl)-2-(1-piperazinyl)-1H-benzimidazole MP:84 to 86°C.
- 1-Ethyl-2-(1-piperazinyl)-1H-benzimidazole. MP (fumarate): 170 to 172°C.
- 1-Propyl-2-(1-piperazinyl)-1H-benzimidazole. MP (fumarate): 175 to 177°C.

### Example 2

### Preparation of 1-Methyl-2-[(4-ethyoxycarbonyl)-1-piperazinyl]-1H-benzimidazole.

A mixture of 3.3 g (20 mmol) of 2-Chloro-1-methyl-1H-benzimidazole and 3.2 g (20 mmol) of 1-Ethoxycarbonylpiperazine was heated for 25 minutes at 120^{º}C. The reagent mixture was provided with a 10% NaOH aqueous solution and extracted with dichloromethane (3 x 20 ml). The organic extracts were gathered, dried with anhydrous sodium sulphate and the solvent was eliminated at diminished pressure. 4.8 g (84% yield) of the product was obtained as a white solid that was purified by column chromatography, using dichloromethane/methanol (95/5) as eluent. MP: 122-4^{º}C.

The following compounds were similarly prepared:
- 1-Methyl-2-[(4-diphenylmethyl)-1-piperazinyl]-1H-benzimidazole. MP (fumarate): 123 to 125°C.
- 1-Phenylmethyl-2-[(4-diphenylmethyl)-1-piperazinyl]-1H-benzimidazole. MP (fumarate) 155 to 158°C.

### Example 3

### Preparation of 1-Methyl-2-[(4-hydroxymethyl)-1-piperazinyl]-1H-benzimidazole.

0.5 g of aluminium and lithium hydride were suspended in 75 ml of dry ether. 4.3 g (15 mmol) of 1-Methyl-2-[(4-ethoxycarbonyl)-1-piperazinyl]-1H-benzimidazole were added slowly, stirring for ten hours at room temperature. This was hydrolized with 20 ml of water and 10 ml of 10% NaOH aqueous solution. The alkaline hydroxides precipitated by filtration were separated and the filtrate extracted with 3 x 20 ml of dichloromethane. The organic extracts were dried with anhydrous sodium sulphate and the solvent was eliminated in a vacuum. 3.4 g of and oil remained, that was purified by column chromatography, using dichloromethane/methanol (9/1) as eluent, obtaining 1.8 g (50% yield) of the above-mentioned product. MP: 82 to 85°C.

## Claims (Claims for the following Contracting State(s): AT, BE, CH, DE, DK, FR, GB, IT, LI, LU, NL)

1. 2-Piperazinylbenzimidazole derivatives having the general formula 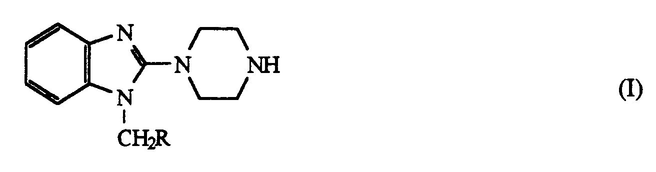 wherein R represents a hydrogen atom, a linear or branched alkyl group having 1 to 3 carbon atoms or a phenyl group which is unsubstituted or substituted by a halogen atom in the para position, as well as their addition salts with pharmaceutically acceptable acids.

2. 2-Piperazinylbenzimidazole derivatives according to claim 1, namely
- 1-Methyl-2-(1-piperazinyl-)1H-benzimidazole or
- 1-Phenylmethyl-2-(1-piperazinyl-)1H-benzimidazole or
- 1-(4-Fluorophenylmethyl-)2-(1-piperazinyl-)1H-benzimidazole or
- 1-Ethyl-2-(1-piperazinyl-)1H-benzimidazole or
- 1-Propyl-2-(1-piperazinyl-)1H-benzimidazole.

3. 2-Piperazinylbenzimidazole derivatives according to claim 1 or claim 2 for use as antagonists to the action of serotonin at the 5HT₃ receptors level.

4. 2-Piperazinylbenzimidazole derivatives according to claim 1 or claim 2 for use as a medicament.

5. 2-Piperazinylbenzimidazole derivatives according to claim 1 or claim 2 for use as a medicament for preventing vomit.

6. 2-Piperazinylbenzimidazole derivatives according to claim 1 or claim 2 for use as a medicament useful in the prophylaxis and treatment of migraine.

7. 2-Piperazinylbenzimidazole derivatives according to claim 1 or claim 2 for use as a medicament useful in the prophylaxis and treatment of anxiety.

8. 2-Piperazinylbenzimidazole derivatives according to claim 1 or claim 2 for use as a medicament useful in the prophylaxis and treatment of neuralgic disorders.

## Claims (Claims for the following Contracting State(s): GR)

1. A process for preparing 2-piperazinylbenzimidazole derivatives having the general formula 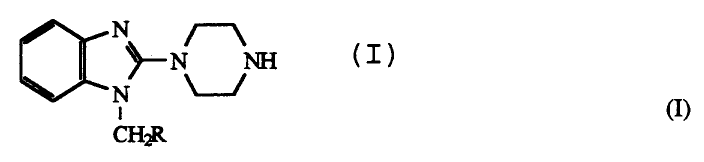 wherein R represents a hydrogen atom, a linear or branched alkyl group having 1 to 3 carbon atoms or a phenyl group which is unsubstituted or substituted by a halogen atom in the para position, as well as their addition salts with pharmaceutically acceptable acids by reacting piperazine with a halogenated benzimidazole derivative for a period of time ranging between 5 and 30 min at a temperature of from 80 to 150 °C.

2. Process according to claim 1, wherein said benzimidazole derivative has the general formula (II) 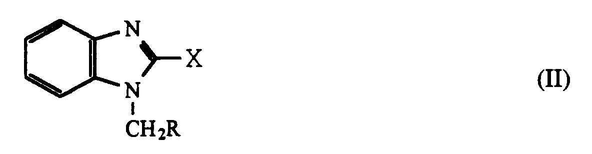 wherein X represents a halogen atom, preferably a chlorine atom, and R is defined as in claim 1.

## Patentansprüche (Patentansprüche für folgende(n) Vertragsstaat(en): AT, BE, CH, DE, DK, FR, GB, IT, LI, LU, NL)

1. 2-Piperazinylbenzimidazol-Derivate der allgemeinen Formel 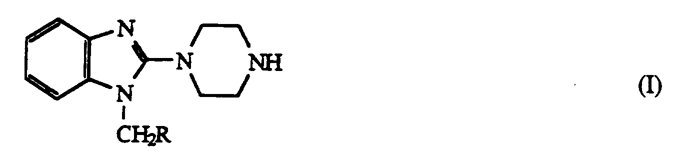 worin R für ein Wasserstoffatom, eine lineare oder verzweigte Alkyl-Gruppe mit 1 bis 3 Kohlenstoffatomen oder eine Phenyl-Gruppe, die unsubstituiert ist oder durch ein Halogenatom in para-Position substituiert ist, steht, sowie ihre Additionssalze mit pharmazeutisch annehmbaren Säuren.

2. 2-Piperazinylbenzimidazol-Derivate nach Anspruch 1, nämlich
- 1-Methyl-2-(1-piperazinyl-)1H-benzimidazol oder
- 1-Phenylmethyl-2-(1-piperazinyl-)1H-benzimidazol oder
- 1-(4-Fluorphenylmethyl-)2-(1-piperazinyl-)1H-benzimidazol oder
- 1-Ethyl-2-(1-piperazinyl-)1H-benzimidazol oder
- 1-Proply-2-(1-piperazinyl-)1H-benzimidazol.

3. 2-Piperazinylbenzimidazol-Derivate nach Anspruch 1 oder Anspruch 2, zur Verwendung als Antagonist zur Wirkung von Serotonin an dem 5HT₃-Rezeptorbereich.

4. 2-Piperazinylbenzimidazol-Derivate nach Anspruch 1 oder Anspruch 2, zur Verwendung als Medikament.

5. 2-Piperazinylbenzimidazol-Derivate nach Anspruch 1 oder Anspruch 2, zur Verwendung als Medikament zur Vorbeugung gegen Erbrechen.

6. 2-Piperazinylbenzimidazol-Derivate nach Anspruch 1 oder Anspruch 2, zur Verwendung als Medikament, das nützlich ist bei der Prophylaxe und Behandlung von Migräne.

7. 2-Piperazinylbenzimidazol-Derivate nach Anspruch 1 oder Anspruch 2, zur Verwendung als Medikament, das nützlich ist bei der Prophylaxe und Behandlung von Angstzuständen.

8. 2-Piperazinylbenzimidazol-Derivate nach Anspruch 1 oder Anspruch 2, zur Verwendung als Medikament, das nützlich ist bei der Prophylaxe und Behandlung von neuralgischen Beschwerden.

## Patentansprüche (Patentansprüche für folgende(n) Vertragsstaat(en): GR)

1. Verfahren zur Herstellung von 2-Piperazinylbenzimidazol-Derivaten der allgemeinen Formel 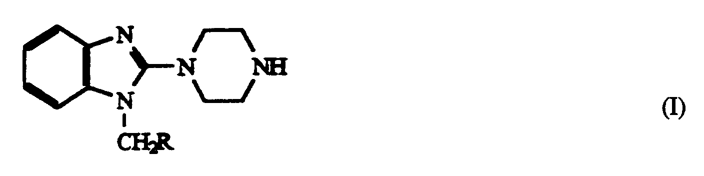 worin R für ein Wasserstoffatom, eine lineare oder verzweigte Alkyl-Gruppe mit 1 bis 3 Kohlenstoffatomen oder eine Phenyl-Gruppe, die unsubstituiert ist oder durch ein Halogenatom in para-Position substituiert ist, steht, sowie ihrer Additionssalze mit pharmazeutisch annehmbaren Säuren durch Umsetzen von Piperazin mit einem halogenierten Benzimidazol-Derivat für eine Zeitdauer im Bereich zwischen 5 und 30 Minuten bei einer Temperatur von 80 bis 150 °C.

2. Verfahren nach Anspruch 1, worin das Benzimidazol-Derivat die allgemeine Formel (II) aufweist: 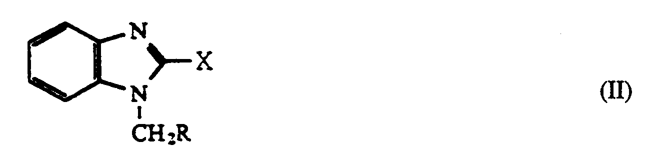 worin X für ein Halogenatom, vorzugsweise ein Chloratom steht, und R wie in Anspruch 1 definiert ist.

## Revendications (Revendications pour l'(les) Etat(s) contractant(s) suivant(s): AT, BE, CH, DE, DK, FR, GB, IT, LI, LU, NL)

1. Dérivés de pipérazinyl-2-benzimidazole répondant à la formule générale : 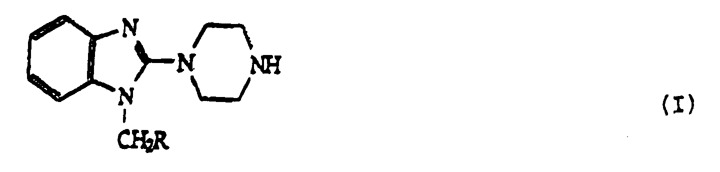
dans laquelle R représente un atome d'hydrogène, un groupe alkyle linéaire ou ramifié de 1 à 3 atomes de carbone, ou un groupe phényle qui est non substitué ou substitué par un atome d'halogène dans la position para,
ainsi que leurs sels d'addition d'acides acceptables au plan pharmaceutique.

2. Dérivés de pipérazinyl-2-benzimidazole selon la revendication 1, à savoir
- 1-méthyl-2-(1-pipérazinyl)-1H-benzimidazole ou
- 1-phénylméthyl-2-(1-pipérazinyl)-1H-benzimidazole ou
- 1-(4-fluorophénylméthyl)-2-(1-pipérazinyl)-1H-benzimidazole ou
- 1-éthyl-2-(1-pipérazinyl)-1H-benzimidazole ou
- 1-propyl-2-(1-pipérazinyl)-1H-benzimidazole.

3. Dérivés de pipérazinyl-2-benzimidazole selon la revendication 1 ou la revendication 2, destinés à être utilisés en tant qu'antagonistes de l'activité de la sérotonine au niveau des récepteurs 5HT₃.

4. Dérivés de pipérazinyl-2-benzimidazole selon la revendication 1 ou la revendication 2 destinée à être utilisés comme un médicament.

5. Dérivés de pipérazinyl-2-benzimidazole selon la revendication 1 ou la revendication 2, destinés à être utilisée comme un médicament anti-émétique.

6. Dérivés de pipérazinyl-2-benzimidazole selon la revendication 1 ou la revendication 2, destinés à être utilisés comme un médicament utile dans la prophylaxie et le traitement de la migraine.

7. Dérivés de pipérazinyl-2-benzimidazole selon la revendication 1 ou la revendication 2, destinés à être utilisés comme un médicament utile dans la prophylaxie et le traitement de l'anxiété.

8. Dérivés de pipérazinyl-2-benzimidazole selon la revendication 1 ou la revendication 2, destinés à être utilisés comme un médicament utile dans la prophylaxie et le traitement des troubles neurologiques.

## Revendications (Revendications pour l'(les) Etat(s) contractant(s) suivant(s): GR)

1. Procédé pour préparer des dérivés de pipérazinyl-2-benzimidazole répondant à la formule générale : 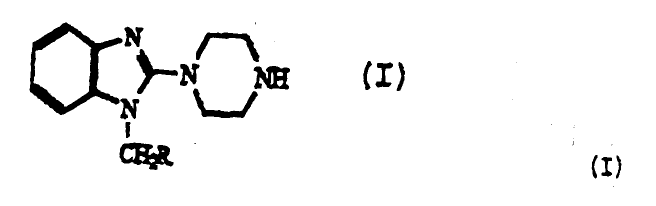
dans laquelle R représente un atome d'hydrogène, un groupe alkyle linéaire ou ramifié de 1 à 3 atomes de carbone, ou un groupe phényle qui est non substitué ou substitué par un atome d'halogène dans la position para,
ainsi que leurs sels d'addition d'acides acceptables au plan pharmaceutique par réaction de la pipérazine avec un dérivé benzimidazole halogéné pendant une période de temps comprise entre 5 et 30 minutes à une température de 80 à 150° C.

2. Procédé selon la revendication 1 dans lequel ledit dérivé benzimidazole a une formule générale (II). 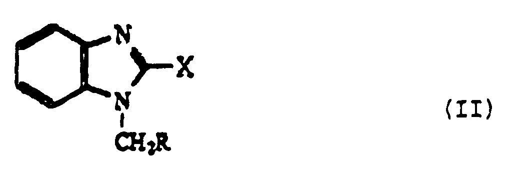 dans laquelle X représente un atome d'halogène, de préférence un atome de chlore, et R comme défini dans la revendication 1.
